# EUROPÄISCHE PATENTSCHRIFT

(11) **EP 0 309 958 B1**
(45) Veröffentlichungstag und Bekanntmachung des Hinweises auf die Patenterteilung: **07.01.1993**
(21) Anmeldenummer: 88115790.3
(22) Anmeldetag: 26.09.1988
(51) Int. Cl.: C07C 17/42, C07C 29/94

(54) **Stabilisierte ungesättigte organische Chlorverbindungen mit C3, deren Gemische oder Zubereitungen**
Stabilized unsaturated chlorine-substituted compounds with C3, and their mixtures
Composés organiques en C3 insaturés stabilisés et substitués par du chlore et leurs mélanges

(30) Priorität: 01.10.1987 DE 3733209
(43) Veröffentlichungstag der Anmeldung: 05.04.1989
(73) Patentinhaber: DEUTSCHE SOLVAY-WERKE GMBH, 42697 Solingen (DE)
(72) Erfinder: Dilla, Wolfgang, Dr. Dipl.-Chem., D-4134 Rheinberg (DE); Jakobson, Gerald, Dr. Dipl.-Chem., D-4134 Rheinberg 2 (DE)
(74) Vertreter: Seiler, Siegfried

(56) Entgegenhaltungen:
- BE-A- 562 288
- BE-A- 624 361
- DE-B- 1 127 346
- US-A- 2 364 588
- US-A- 2 935 537
- CHEMICAL ABSTRACTS, Band 78, 1973, Seite 422, Nr. 110527v, Columbus, Ohio, US; & JP-A-72 39 011
- IND. ENG. CHEM. PROD. RES. DEV., vol. 18, no. 2, 1979, pages 131-135, American Chemical Society; W. L. ARCHER, "Comparison of chlorinated solvent-aluminum reaction inhibitors"

## Beschreibung

Die Erfindung betrifft stabilisierte ungesättigte organische Chlorverbindungen mit C₃, deren Gemische oder Zubereitungen. Ungesättigte organische Chlorverbindungen mit C₃, wie ungesättigte chlorierte Kohlenwasserstoffe mit C₃, zersetzen sich unter der Einwirkung von Wärme, Licht, Luftsauerstoff, Verunreinigungen, Metallen und Metallsalzen.

Der Abbau der genannten Verbindungen äußert sich in einer Verfärbung, Zunahme des Chloridgehaltes und der Acidität sowie in der Bildung von Oligomeren und damit in einer Erhöhung des Abdampfrückstandes. Metallische Werkstoffe werden korrodiert.

Zur Erhaltung der Produktqualität sowie zur Vermeidung von Korrosionsschäden ist daher eine Stabilisierung der genannten ungesättigten organischen Chlorverbindungen mit C₃ erforderlich.

Aus US-A-23 64 588 ist die Stabilisierung von 2-Clorbuten-2 durch Zugabe von 1 bis 5 Vol% einer Epoxiverbindung bekannt.

In US-A-29 35 537 und BE-A-562 288 wird die Stabilisierung von Trichloräthylen und Perchloräthylen durch Zusatz von Phenol und Epichlorhydrin und Butylenoxid beschrieben.

Aus BE-A-624 361 ist die Stabilisierung von Trichloräthylen durch Zugabe von Epoxyverbindung und Phenol bekannt.

Aus DE-B-11 27 346 ist die Stabilisierung von Trichloräthylen und Tetrachloräthylen durch Zugabe von Phenolen und gesättigten Aliphatischen Alkoholen bekannt.

In Japan Kokai 7239,011 vom 6.2.72 zitiert in Chemical Abstracts Vol. 78, 1973, S. 422, Abstract-Nr. 110 527, wird eine Methode zur Stabilisierung von Methallylchlorid mit 500 mg/kg 2,6 -Di.-tert.-butyl-4-methyl-phenol beschrieben. Durch diese Stabilisierung wird die Oligomerisierung und Verfärbung verhindert. Nachteilig an diesem Verfahren ist der hohe Gehalt des Stabilisators, der in der angegebenen Konzentration zur Korrosion von z. B. Eisen führen kann. Weiterhin wird durch den Stabilisator die direkte Reaktion des Chlorkohlenwasserstoffes mit dem metallischen Werkstoff nicht unterbunden. Entstehende Metallchloride oder Chlorwasserstoff werden nicht inaktiviert.

In der Literaturstelle Revista de Chimie 1973, 24(4), S. 241-246 wird Hydrochinon als geeigneter Stabilisator für 2,3-Dichlorpropen angegeben. Bei eigenen Untersuchungen hat sich dieser Stabilisator sowohl im Hinblick auf die Unterbindung der Verfärbung des Produktes als auch bei der Chloridbildung als ungeeignet bzw. als wenig wirksam erwiesen. Das weiterhin angegebene Triethanolamin hat den Nachteil, daß mit Chlorwasserstoff ein Aminhydrochlorid gebildet werden kann. Dieses Aminhydrochlorid kann bei Zutritt von Wasser wieder ionisches Chlorid abspalten. Aus Triethanolaminhydrochlorid kann ferner durch Stahlkorrosion Wasserstoff gebildet werden (C.Kyriazis, E. Heitz, Werkstoffe und Korrosion 1980, 31, 197 - 207).

Das weiterhin geprüfte Propylenoxid hat den Nachteil des vergleichsweise hohen Dampfdruckes und der somit schlechten Handhabbarkeit. Das weiterhin geprüfte Anilin neigt ebenso wie Triethanolamin zur Bildung von Aminhydrochloriden. Ferner besteht die Möglichkeit einer direkten Reaktion des Anilins mit der zu stabilisierenden Verbindung.

Gemäß der rumänischen Patentanmeldung RO 88,609, referiert in C.A. 1987, Vol. 106, S. 485, Abstract-Nr. 32349U verwendet man 2-sec.-butyl-4,6-di-tert.-butyl-phenol, Epichlorhydrin und epoxidiertes Sojabohnenöl als Stabilisatoren für 2-(2-chlorethoxy)ethanol. Das epoxidierte Sojabohnenöl hat den Nachteil der Eigenfärbung und der vergleichsweise geringen Wirkung.

Das in der CA-PS 407,152 beschriebene Verfahren zur Stabilisierung von Allylhalogeniden besteht aus einem Zusatz von Wasser. Dieses Verfahren ist nicht immer praktikabel, insbesondere dann nicht, wenn höhere Anforderungen an die Produktreinheit gestellt werden.

Zur Stabilisierung von Nematiziden, die Dichlorpropen enthalten, werden gemäß Japan Kokai Tokkyo Koho 57126429 vom 29.1.81, zitiert in Chemical Abstracts Vol. 98, Abstract-Nr. 12946, organische Zinnverbindungen vorgeschlagen.

Der Erfindung liegt die Aufgabe zugrunde, die vorgenannten Nachteile zu vermeiden und einen Stabilisator oder ein Stabilisatorgemisch zur Stabilisierung der ungesättigten chlorierten Kohlenwasserstoffe mit C₃ zur Verfügung zu stellen, mit dessen Hilfe eine wirksame Stabilisierung dieser Verbindungen bei längerer Lagerzeit, auch in Gegenwart metallischer Werkstoffe, erreicht wird. Es sollte keine Verfärbung oder eine andere nachteilige Veränderung beobachtet werden.

Zur Lösung der Aufgabe enthalten die stabilisierten ungesättigten organischen Chlorverbindungen mit C₃, deren Gemische oder Zubereitungen erfindungsgemäß Stabilisatorgemisch, bestehend aus
5 - 150 mg/kg, vorzugsweise
10 - 100 mg/kg,
bezogen auf ungesättigte chlorierte Kohlenwasserstoffe) mindestens eines Phenolderivates aus 4-Tert.-butyl-phenol, 2-Tert.-butyl-4-methyl-phenol, 2,4-Di-tert.-butyl-phenol, 2,6-Di-tert.-butyl-4-methyl-phenol, 2,4,6-Tri-methylphenol, 2-Isopropyl-5-methylphenol, Pentaerythrityltetrakis-3(3-(3,5-di-tert.-butyl-4-hydroxyphenyl)-propionat), N,N'-(Hexamethylen-bis-(3,5-di-tert.-butyl-4-hydroxyphenyl)-propionat), N,N'-(Hexamethylen-bis-(3,5-di-tert.-butyl-4-hydroxy-hydrozimt-säureamid) oder Octadecyl-3-(3,5-di-tert.-butyl-4-hydroxy-phenyl)-propionat oder zwei oder mehreren dieser Verbindungen und aus mindestens einer der folgenden Gruppen A und B
A)
   100 - 20.000 mg/kg,vorzugsweise
   500 - 5.000 mg/kg,
   (bezogen auf ungesättigte chlorierte Kohlenwasserstoffe)mindestens einer epoxygruppenhaltigen Verbindung aus 1,2-Epoxybutan, 1,2-Epoxyhexan, 1,2-Epoxyoctan, 2,3-Epoxypropylisopropylether oder 2,2-Dimethyl-4(2',3'-epoxy)-propoxymethyl1,3-dioxolan oder mehreren dieser Verbindungen,
B)
   100 - 30.000 mg/kg,vorzugsweise
   500 - 20.000 mg/kg,
   (bezogen auf ungesättigte chlorierte Kohlenwasserstoffe) eines tertiären Alkohols aus 2-Methyl-2-propanol oder 2-Methyl-2-butanol oder beide Verbindungen, wobei das Gewichtsverhältnis von Phenolderivat zu Epoxyverbindungen und/oder zu dem tertiären Alkohol
   1 : 10 bis 1 : 6000, vorzugsweise
   1 : 50 bis 1 : 500
   beträgt.

Die zu stabilisierenden ungesättigten organischen Chlorverbindungen mit C₃, deren Gemische oder Zubereitungen bestehen aus den ungesättigten chlorierten Kohlenwasserstoffen 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen oder 1,2,3-Trichlor-1-propen oder enthalten eine oder mehrere dieser Verbindungen.

Gemäß einer vorzugsweisen Ausführungsform enthalten 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen oder 1,2,3-Trichlor-1-propen ein Stabilisatorgemisch bestehend aus
5 - 150 mg/kg, vorzugsweise
10 - 100 mg/kg,
(bezogen auf den ungesättigten chlorierten Kohlenwasserstoff) 2,6-Di-tert.-butyl-4-methyl-phenol und
100 - 20.000 mg/kg, vorzugsweise
500 - 5.000 mg/kg,
(bezogen auf den ungesättigten chlorierten Kohlenwasserstoff) 1,2-Epoxybutan und
100 - 30.000 mg/kg, vorzugsweise
500 - 20.000 mg/kg,
(bezogen auf den ungesattigten chlorierten Kohlenwasserstoff) 2-Methyl-2-propanol, wobei das Gewichtsverhältnis von Phenolderivat zu Epoxyverbindungen und zu dem tertiären Alkohol
1 : 10 bis 1 : 6000, vorzugsweise
1 : 50 bis 1 : 500,
beträgt.

Gemäß einer weiteren vorteilhaften Ausführungsform enthalten 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen oder 1,2,3-Trichlor-1-propen ein Stabilisatorgemisch, bestehend aus
5 - 150 mg/kg, vorzugsweise
10 - 100 mg/kg,
(bezogen auf den ungesättigten chlorierten Kohlenwasserstoff) 2,6-Di-tert.-butyl-4-methyl-phenol und
100 - 20.000 mg/kg,vorzugsweise
500 - 5.000 mg/kg,
(bezogen auf den ungesättigten chlorierten Kohlenwasserstoff) 2,2-Dimethyl-4-(2',3'-epoxy)-propoxymethyl-1,3-dioxolan, wobei das Gewichtsverhältnis von Phenolderivat zu Epoxyverbindung
1 : 10 bis 1 : 6000, vorzugsweise
1 : 50 bis 1 : 500
beträgt.

Die erfindungsgemäß stabilisierten ungesättigten chlorierten Kohlenwasserstoffe mit C₃ werden als Ausgangsprodukte zur Herstellung von Medikamenten, Pflanzenschutzmitteln, Farbstoffen, Haftvermittlern und Weichmachern verwendet.

### Beispiel 1

2,3-Dichlor-1-propen wurde unter Zusatz von 50 mg/kg 2,6-Di-tert.-butyl-4-methyl-phenol und 5000 mg/kg 1,2-Epoxybutan in Gegenwart des Edelstahls 1.4571 24 Stunden unter Rückfluß (93 °C) erhitzt. Nach dieser Zeit betrug die Farbe des 2,3-Dichlor-1- propens 40 APHA und der Chloridgehalt 29 mg/kg. Ohne Zusatz der Stabilisatoren, unter sonst gleichen Versuchsbedingungen, betrug die Farbe des 2,3Dichlor-1propens mehr als 150 APHA und der Chloridgehalt 110 mg/kg.

### Beispiel 2

2,3-Dichlor-1-propen wurde unter Zusatz von 50 mg/kg 2,6-Di-tert.-butyl-4-methyl-phenol und 5000 mg/kg 2,2-Dimethyl-4-(2',3'-epoxy)-propoxy-methyl-1,3-dioxolan in Gegenwart des Kohlenstoffstahls St.37 vier Wochen lang bei Raumtemperatur gelagert. Nach dieser Zeit betrug die Farbe des 2,3-Dichlor-1-propens 10 APHA, der Chloridgehalt 10 mg/kg. Ein Massenverlust des Kohlenstoffstahls war nicht nachzuweisen. Ohne Zusatz der Stabilisatoren, unter sonst gleichen Versuchsbedingungen, betrug die Farbe des 2,3-Dichlor-1-propens mehr als 150 APHA, der Chloridgehalt 490 mg/kg und die Abtragsrate des Kohlenstoff stahls 0,05 mm/a.

### Beispiel 3

1,3-Dichlor-1-propen wurde unter Zusatz von 50 mg/kg 2,6-Di-tert.-butyl-4-methyl-phenol und 10000 mg/kg 2-Methyl-2-propanol in Gegenwart des Kohlenstoffstahls St.37 vier Wochen lang bei Raumtemperatur gelagert. Nach dieser Zeit betrug die Farbe des 1,3-Dichlor-1-propens 25 APHA, der Chloridgehalt 3 mg/kg und der Abdampfrückstand 0,07 g/kg Ohne Zusatz der Stabilisatoren, unter sonst gleichen Versuchsbedingungen, betrug die Farbe 50 APHA, der Chloridgehalt 50 mg/kg und der Abdampfrückstand 4,85 g/kg.

## Patentansprüche

1. Stabilisierte ungesättigte organische Chlorverbindungen mit C₃, deren Gemische oder Zubereitungen, dadurch gekennzeichnet, daß sie ein Stabilisatorgemisch, bestehend aus
5 - 150 mg/kg, vorzugsweise
10 - 100 mg/kg,
(bezogen auf ungesättigte chlorierte Kohlenwasserstoffe) mindestens eines Phenolderivates aus 4-Tert.-butyl-phenol, 2-Tert.-butyl-4-methyl-phenol, 2,4-Di-tert.-butyl-phenol, 2,6-Di-tert.-butyl-4-methyl-phenol, 2,4,6-Tri-methylphenol, 2-Isopropyl-5-methylphenol, Pentaerythrityl-tetrakis-3(3-(3,5-di-tert.-butyl-4-hydroxyphenyl)-propionat), N,N'-(Hexamethylen-bis-(3,5-di-tert.-butyl-4-hydroxy-phenyl)-propionat), N,N'-(Hexamethylen-bis-(3,5-di-tert.-butyl-4-hydroxy-hydrozimt-säureamid) oder Octadecyl-3-(3,5-di-tert.-butyl-4-hydroxy-phenyl)-propionat oder zwei oder mehreren dieser Verbindungen und aus mindestens einer der folgenden Gruppen A und B
A)
100 - 20.000 mg/kg,vorzugsweise
500 - 5.000 mg/kg,
(bezogen auf ungesättigte chlorierte Kohlenwasserstoffe) mindestens einer epoxygruppenhaltigen Verbindung aus 1,2-Epoxybutan, 1,2-Epoxyhexan, 1,2-Epoxyoctan, 2,3-Epoxypropylisopropylether oder 2,2-Dimethyl-4(2',3'-epoxy)-propoxymethyl-1,3-dioxolan oder mehreren dieser Verbindungen,
B)
100 - 30.000 mg/kg,vorzugsweise
500 - 20.000 mg/kg,
(bezogen auf ungesättigte chlorierte Kohlenwasserstoffe) eines tertiären Alkohols aus 2-Methyl-2-propanol oder 2-Methyl-2-butanol oder beide Verbindungen enthalten, wobei das Gewichtsverhältnis von Phenolderivat zu Epoxyverbindungen und/oder zu dem tertiären Alkohol
1 : 10 bis 1 : 6000, vorzugsweise
1 : 50 bis 1 : 500
beträgt.

2. Stabilisierte ungesättigte organische Chlorverbindungen mit C₃, deren Gemische oder Zubereitungen, dadurch gekennzeichnet, daß sie aus den ungesättigten chlorierten Kohlenwasserstoffen 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen oder 1,2,3-Trichlor-1-propen bestehen oder eine oder mehrere dieser Verbindungen enthalten.

3. Stabilisierte ungesättigte organische Chlorverbindungen mit C₃, deren Gemische oder Zubereitungen nach Ansprüchen 1 und 2, dadurch gekennzeichnet, daß 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen, oder 1,2,3-Trichlor-1-propen ein Stabilisatorgemisch, bestehend aus
5 - 150 mg/kg, vorzugsweise
10 - 100 mg/kg,
(bezogen auf den ungesättigten chlorierten Kohlenwasserstoff) 2,6-Di-tert.-butyl-4-methylphenol und
100 - 20.000 mg/kg,vorzugsweise
500 - 5.000 mg/kg,
(bezogen auf den ungesättigten chlorierten Kohlenwasserstoff) 1,2-Epoxybutan und
100 - 30.000 mg/kg, vorzugsweise
500 - 20.000 mg/kg,
(bezogen auf den ungesättigten chlorierten Kohlenwasserstoff) 2-Methyl-2-propanol, enthalten, wobei das Gewichtsverhältnis von Phenolderivat zu Epoxyverbindungen und zu dem tertiären Alkohol
1 : 10 bis 1 : 6000, vorzugsweise
1 : 50 bis 1 : 500,
beträgt.

4. Stabilisierte ungesättigte organische Chlorverbindungen mit C₃, deren Gemische oder Zubereitungen nach Ansprüchen 1 und 2, dadurch gekennzeichnet, daß 1-Chlor-1-propen, 2-Chlor-1-propen, 3-Chlor-1-propen, 1,3-Dichlor-1-propen, 2,3-Dichlor-1-propen, 3,3-Dichlor-1-propen, 1,2-Dichlor-1-propen, 1,1-Dichlor-1-propen, 3,3,3-Trichlor-1-propen, 2,3,3-Trichlor-1-propen, 1,3,3-Trichlor-1-propen, 1,1,3-Trichlor-1-propen, 1,1,2-Trichlor-1-propen oder 1,2,3-Trichlor-1-propen ein Stabilisatorgemisch, bestehend aus
5 - 150 mg/kg, vorzugsweise
10 - 100 mg/kg,
(bezogen auf den ungesättigten chlorierten Kohlenwasserstoff) 2,6-Di-tert.-butyl-4-methylphenol und
100 - 20.000 mg/kg,vorzugsweise
500 - 5.000 mg/kg,
(bezogen auf den ungesättigten chlorierten Kohlenwasserstoff) 2,2-Dimethyl-4-(2',3'-epoxy)-propoxymethyl-1,3-dioxolan enthalten, wobei das Gewichtsverhältnis von Phenolderivat zu Epoxyverbindung
1 : 10 bis 1 : 6000, vorzugsweise
1 : 50 bis 1 : 500
beträgt.

5. Verwendung der gemäß Ansprüchen 1 bis 4 stabilisierten ungesättigten chlorierten Kohlenwasserstoffe mit C₃ als Ausgangsprodukte zur Herstellung von Medikamenten, Pflanzenschutzmitteln, Farbstoffen, Haftvermittlern und Weichmachern.

## Claims

1. Stabilised, unsaturated, organic chlorine compounds with C₃, mixtures or preparations thereof, characterised in that they contain a stabiliser mixture consisting of
5 - 150 mg/kg, preferably
10 - 100 mg/kg,
(relative to unsaturated, chlorinated hydrocarbons) of at least one phenol derivative from 4-tert.-butyl-phenol, 2-tert.-butyl-4-methyl-phenol, 2,4-di-tert.-butyl-phenol, 2,6-di-tert.-butyl-4-methyl-phenol, 2,4,6-tri-methylphenol, 2-isopropyl-5-methylphenol, pentaerithrityl-tetrakis-3(3-(3,5-di-tert.-butyl-4-hydroxyphenyl)-propionate), N,N-(hexamethylene-bis-(3,5-di-tert.-butyl-4-hydroxyphenyl)-propionate), N,N'-(hexamethylene-bis-(3,5-di-tert.-butyl-4-hydroxy-hydrocinnamic acid amide) or octadecyl-3-(3,5-di-tert.-butyl-4-hydroxy-phenyl)-propionate or two or more of these compounds and of at least one of the following groups A and B
A)
100 - 20,000 mg/kg, preferably
500 - 5,000 mg/kg,
(relative to unsaturated, chlorinated hydrocarbons) of at least one compound containing epoxy groups from 1,2-epoxybutane, 1,2-epoxyhexane, 1,2-epoxyoctane, 2,3-epoxypropylisopropylether or 2,2-dimethyl-4(2',3'-epoxy)-propoxymethyl-1,3-dioxolane or several of these compounds,
B)
100 - 30,000 mg/kg, preferably
500 - 20,000 mg/kg,
(relative to unsaturated, chlorinated hydrocarbons) of a tertiary alcohol from 2-methyl-2-propanol or 2-methyl-2-butanol or both compounds,
the weight ratio of phenol derivative to epoxy compounds and/or to the tertiary alcohol being
1 : 10 to 1 : 6000, preferably
1 : 50 to 1 : 500.

2. Stabilised, unsaturated, organic chlorine compounds with C₃, mixtures or preparations thereof, characterised in that they consist of the unsaturated, chlorinated hydrocarbons 1-chloro-1-propene, 2-chloro-1-propene, 3-chloro-1-propene, 1,3-dichloro-1-propene, 2,3-dichloro-1-propene, 3,3-dichloro-1-propene, 1,2-dichloro-1-propene, 1,1-dichloro-1-propene, 3,3,3-trichloro-1-propene, 2,3,3-trichloro-1-propene, 1,3,3-trichloro-1-propene, 1,1,3-trichloro-1-propene, 1,1,2-trichloro-1-propene or 1,2,3-trichloro-1-propene or contain one or more of these compounds.

3. Stabilised, unsaturated, organic chlorine compounds with C₃, mixtures or preparations thereof according to Claims 1 and 2, characterised in that 1-chloro-1-propene, 2-chloro-1-propene, 3-chloro-1-propene, 1,3-dichloro-1-propene, 2,3-dichloro-1-propene, 3,3-dichloro-1-propene, 1,2-dichloro-1-propene, 1,1-dichloro-1-propene, 3,3,3-trichloro-1-propene, 2,3,3-trichloro-1-propene, 1,3,3-trichloro-1-propene, 1,1,3-trichloro-1-propene, 1,1,2-trichloro-1-propene or 1,2,3-trichloro-1-propene contain a stabiliser mixture consisting of
5 - 150 mg/kg, preferably
10 - 100 mg/kg,
(relative to the unsaturated chlorinated hydrocarbon) 2,6-di-tert.-butyl-4-methylphenol and
100 - 20,000 mg/kg, preferably
500 - 5,000 mg/kg,
(relative to the unsaturated chlorinated hydrocarbon) 1,2-epoxybutane and
100 - 30,000 mg/kg, preferably
500 - 20.000 mg/kg,
(relative to the unsaturated chlorinated hydrocarbon) 2-methyl-2-propanol, the weight ratio of phenol derivative to epoxy compounds and to the tertiary alcohol being
1 : 10 to 1 : 6000, preferably
1 : 50 to 1 : 500.

4. Stabilised, unsaturated, organic chlorine compounds with C₃, mixtures or preparations thereof according to Claims 1 and 2, characterised in that 1-chloro-1-propene, 2-chloro-1-propene, 3-chloro-1-propene, 1,3-dichloro-1-propene, 2,3-dichloro-1-propene, 3,3-dichloro-1-propene, 1,2-dichloro-1-propene, 1,1-dichloro-1-propene, 3,3,3-trichloro-1-propene, 2,3,3-trichloro-1-propene, 1,3,3-trichloro-1-propene, 1,1,3-trichloro-1-propene, 1,1,2-trichloro-1-propene or 1,2,3-trichloro-1-propene contain a stabiliser mixture consisting of
5 - 150 mg/kg, preferably
10 - 100 mg/kg,
(relative to the unsaturated chlorinated hydrocarbon) 2,6-di-tert.-butyl-4-methylphenol and
100 - 20,000 mg/kg, preferably
500 - 5,000 mg/kg,
(relative to the unsaturated, chlorinated hydrocarbon) 2,2-dimethyl-4-(2',3'-epoxy)-propoxymethyl-1,3-dioxolane,
the weight ratio of phenol derivative to epoxy compound being
1 : 10 to 1 : 6000, preferably
1 : 50 to 1 : 500.

5. The use of the stabilised, unsaturated, chlorinated hydrocarbons with C₃ according to Claims 1 to 4 as starting products for the production of medicaments, plant-protection agents, dyes, adhesion promoters and softeners.

## Revendications

1. Composés organiques chlorés en C₃ insaturés et stabilisés, leurs mélanges ou leurs préparations, caractérisés en ce qu'ils contiennent un mélange de stabilisateurs constitué de :
5 - 150 mg/kg, de préférence
10 - 100 mg/kg,
(par rapport aux hydrocarbures chlorés insaturés) d'au moins un dérivé phénolique constitué de 4-tert.-butyl-phénol, de 2-tert.-butyl-4-méthyl-phénol, de 2,4-di-tert.-butyl-phénol, de 2,6-di-tert.-butyl-4-méthyl-phénol, de 2,4,6-tri-méthylphénol, de 2-isopropyl-5-méthylphénol, de pentaérythrityl-tétrakis-3(3-(3,5-tert.-butyl-4-hydroxyphényl)-propionate), de N,N'-(hexaméthylène-bis-(3,5-di-tert.-butyl-4-hydroxy phényl)-propionate), d'amide d'acide N,N'-(hexaméthylène -bis-(3,5-di-tert.-butyl-4-hydroxy-hydrocinnamique) ou d'octadécyl-3-(3,5-di-tert.-butyl-4-hydroxy-phényl)-propionate, ou de deux ou plusieurs de ces dérivés et d'au moins un des groupes A et B suivants :
A)
100 - 20.000 mg/kg, de préférence
500 - 5.000 mg/kg,
(par rapport aux hydrocarbures chlorés insaturés) d'au moins un composé contenant des groupes époxy constitué de 1,2-époxybutane, de 1,2-époxyhexane, de 1,2-époxyoctane, de 2,3-époxypropylisopropyléther ou de 2,2-diméthyl-4(2',3'-époxy)-propoxyméthyl-1,3-dioxolane ou de plusieurs de ces composés,
B)
100 - 30.000 mg/kg, de préférence
500 - 20.000 mg/kg,
(par rapport aux hydrocarbures chlorés insaturés) d'un alcool tertiaire constitué de 2-méthyl-2-propanol ou de 2-méthyl-2-butanol ou des deux composés, le rapport pondéral du dérivé phénolique aux composés époxydés et/ou à l'alcool tertiaire étant de :
1 : 10 à 1: 6.000, de préférence
1 : 50 à 1: 500.

2. Composés organiques chlorés en C₃ insaturés stabilisés, leurs mélanges ou leurs préparations, caractérisés en ce qu'ils sont constitués des hydrocarbures chlorés insaturés suivants : 1-chloro-1-propène, 2-chloro-1-propène, 3-chloro-1-propène, 1,3-dichloro-1-propène, 2,3-dichloro-1-propène, 3,3-dichloro-1-propène, 1,2-dichloro-1-propène, 1,1-dichloro-1-propène, 3,3,3-trichloro-1-propène, 2,3,3-trichloro-1-propène, 1,3,3-trichloro-1-propène, 1,1,3-trichloro-1-propène, 1,1,2-trichloro-1-propène ou 1,2,3-trichloro-1-propène ou contiennent un ou plusieurs de ces composés.

3. Composés organiques chlorés en C₃ insaturés stabilisés, leurs mélanges ou leurs préparations, caractérisés en ce que les hydrocarbures 1-chloro-1-propène, 2-chloro-1-propène, 3-chloro-1-propène, 1,3-dichloro-1-propène, 2,3-dichloro-1-propène, 3,3-dichloro-1-propène, 1,2-dichloro-1-propène, 1,1-dichloro-1-propène, 3,3,3-trichloro-1-propène, 2,3,3-trichloro-1-propène, 1,3,3-trichloro-1-propène, 1,1,3-trichloro-1-propène, 1,1,2-trichloro-1-propène ou 1,2,3-trichloro-1-propène contiennent un mélange de stabilisants constitué de :
5 - 150 mg/kg, de préférence
10 - 100 mg/kg,
(par rapport aux hydrocarbures chlorés insaturés) de 2,6-di-tert.-butyl-4-méthyl-phénol et de :
100 - 20.000 mg/kg, de préférence
500 - 5.000 mg/kg,
(par rapport aux hydrocarbures chlorés insaturés) de 1,2-époxybutane et de :
100 - 30.000 mg/kg, de préférence
500 - 20.000 mg/kg,
(par rapport aux hydrocarbures chlorés insaturés) de 2-méthyl-2-propanol, le rapport pondéral du dérivé phénolique aux composés époxydés et à l'alcool tertiaire étant de :
1 : 10 à 1 : 6.000, de préférence
1 : 50 à 1 : 500.

4. Composés organiques chlorés en C₃ insaturés stabilisés, leurs mélanges ou leurs préparations selon les revendications 1 et 2, caractérisés en ce que les hydrocarbures 1-chloro-1-propène, 2-chloro-1-propène, 3-chloro-1- propène, 1,3-dichloro-1-propène, 2,3-dichloro-1-propène, 3,3-dichloro-1-propène, 1,2-dichloro-1-propène, 1,1-dichloro-1-propène, 3,3,3-trichloro-1-propène, 2,3,3-trichloro-1-propène, 1,3,3-trichloro-1-propène, 1,1,3-trichloro-1-propène, 1,1,2-trichloro-1-propène ou 1,2,3-trichloro-1-propène contiennent un mélange de stabilisants constitué de :
5 - 150 mg/kg, de préférence
10 - 100 mg/kg,
(par rapport aux hydrocarbures chlorés insaturés) de 2,6-di-tert.-butyl-4-méthyl-phénol et de :
100 - 20.000 mg/kg, de préférence
500 - 5.000 mg/kg,
(par rapport aux hydrocarbures chlorés insaturés) de 2,2-diméthyl-4-(2',3'-époxy)-propoxyméthyl-1,3-dioxolane, le rapport pondéral du dérivé phénolique au composé époxydé étant de :
1 : 10 à 1 : 6.000, de préférence
1 : 50 à 1 : 500.

5. Emploi des hydrocarbures chlorés en C₃ insaturés stabilisés selon les revendications 1 à 4 comme produits de départ pour la production de médicaments, de produits de protection végétale, de colorants, d'agents adhésifs ou de plastifiants.
